# EUROPÄISCHE PATENTSCHRIFT

(11) **EP 1 568 352 B1**
(45) Veröffentlichungstag und Bekanntmachung des Hinweises auf die Patenterteilung: **12.03.2008**
(21) Anmeldenummer: 05100763.1
(22) Anmeldetag: 04.02.2005
(51) Int. Cl.: A61Q 5/06, A61K 8/73, A61K 8/81

(54) **Polymerkombination für Stylingmittel**
Polymer combination for styling compositions
Combination de polymères pour des compositions de mise en forme des cheveux

(30) Priorität: 26.02.2004 DE 102004009158
(43) Veröffentlichungstag der Anmeldung: 31.08.2005
(73) Patentinhaber: Beiersdorf AG, 20253 Hamburg (DE)
(72) Erfinder: Detert, Marion, 22455 Hamburg (DE); Otto, Sebastian, 20253 Hamburg (DE)

(56) Entgegenhaltungen:
- EP-A- 0 825 200
- WO-A-00/39176
- WO-A-20/04024779
- DE-A1- 4 422 593
- US-A1- 2003 202 953
- PATENT ABSTRACTS OF JAPAN Bd. 2000, Nr. 21, 3. August 2001 (2001-08-03) & JP 2001 106614 A (NARIS COSMETICS CO LTD), 17. April 2001 (2001-04-17)
- PATENT ABSTRACTS OF JAPAN Bd. 1999, Nr. 03, 31. März 1999 (1999-03-31) & JP 10 330227 A (KOSE CORP), 15. Dezember 1998 (1998-12-15)
- PATENT ABSTRACTS OF JAPAN Bd. 2000, Nr. 05, 14. September 2000 (2000-09-14) & JP 2000 034212 A (KOSE CORP), 2. Februar 2000 (2000-02-02)

## Beschreibung

Die vorliegende Erfindung betrifft Stylingmittel enthaltend eine Kombination aus einem oder mehreren nichtionischen, verdickenden Polymeren, kationischen Polymeren welche gebildet werden aus 2-(N, N-dimethylamino)ethylmethacrylat, Ethylacrylat, Beheneth-25 methacrylat, Ethern aus Allylalkohol und einem Polyethylenglycol/Polypropylenglycol-Ether, Triethylenglycoldi-methacrylat und 2-Hydroxyethylmethacrylat sowie fixierenden Polymeren gewählt aus der Gruppe der nichtionischen und/oder kationischen Polymere und deren Verwendung.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Natürliches Haar hängt meist schlaff und ohne Volumen vom Kopf herab. Ein Ziel der Haarpflege ist es daher, dem Haar Fülle und Frisur zu geben. Zur temporären Verformung des Haares und Gestaltung (engl. Styling) vielseitiger Frisuren verwendet man Stylingmittel (Haarfestiger), meist in Form von Schaumfestigern, Haargelen oder Haarsprays.

Schaumfestiger (auch Haarschaumfestiger genannt) werden in der Regel zur Frisurgestaltung im feuchten Haar verteilt, Haarsprays zur Fixierung auf die (trockene) fertig gestylte Frisur gesprüht. Neben den wegen ihrer leichten und angenehmen Anwendbarkeit zunehmend beliebten Schaumhaarfestigern und flüssigen Haarfestigern werden auch Haarfestigergele (Haargele) angeboten.

Die beiden Grundbausteine von Haarfestigern sind die Filmbildner (d.h. fixierende Polymere) und das Lösungsmittel. Als Filmbildner werden meist nichtionische, anionische, kationische oder amphothere Polymere eingesetzt. Filmbildner bleiben nach dem Verdunsten des Lösungsmittels (meist Wasser und/oder Ethanol) als Film auf dem Haar und fixieren dessen Form. An den auf dem Haar abgelagerten Film werden eine Vielzahl an Anforderungen gestellt: Er soll klar, feuchtigkeitsunempfindlich, festigend, stabil und nicht klebrig sein sowie sich leicht durch handelsübliche Haarshampoos auswaschen lassen. Neben den Filmbildnern enthalten Stylingzubereitungen Verdickungsmittel, die dem Produkt die gewünschte Konsistenz geben.

Herkömmliche Haarfestiger, insbesondere Haargele, haben eine Reihe von Nachteilen. So weisen die in ihnen enthaltenden Filmbildner zwar meist eine hohe Festigungsleistung auf, doch sind die Filme auf dem Haar in der Regel spröde und hinterlassen insbesondere beim Auskämmen Rückstände auf Haar und Kleidung. Diese Rückstände, auch Filmplaken genannt, verleihen dem Anwender ein ungepflegtes Aussehen. Es entsteht der Eindruck, der Anwender leide unter Schuppen-Kopfhaut. Dieses Phänomen tritt insbesondere dann (und insbesondere bei Gelen) auf, wenn kationische Verdickungsmittel eingesetzt werden. Werden anionische Verdickungsmittel eingesetzt, können die Zubereitungen darüber hinaus keine kationischen Pflegestoffe (beispielsweise polyquaternierte Verbindungen) enthalten, was aus Gründen der Haarpflege durchaus wünschenswert wäre.

Es war daher die Aufgabe der vorliegenden Erfindung die Mängel des Standes der Technik zu beseitigen und Stylingzubereitungen zu entwickeln, deren Filmplakenbildung deutlich reduziert ist und in die sich bei Bedarf kationische Pflegesubstanzen einarbeiten lassen.

Überraschend gelöst wird die Aufgabe durch Stylingmittel enthaltend eine Kombination aus
a) einem oder mehreren nichtionischen, verdickenden Polymeren, welche gewählt werden aus der Gruppe der Verbindungen der Cellulosen, der Cellulosederivate und der Guar Gummis,
b) einem kationischen Polymer, welches gebildet wird aus
   31-40 Gew.-% 2-(N, N-dimethylamino)ethylmethacrylat,
   52-62 Gew.-% Ethylacrylat,
   2,5-5 Gew.-% Beheneth-25 methacrylat,
   2-7 Gew.-% eines Ethers aus Allylalkohol und einem Polyethylenglycol/Polypropylenglycol-Ether mit durchschnittlich 30 Ethylenglycol und durchschnittlich 4 Polypropylenglycol-Einheiten,
   0,05-0,2 Gew.-% Triethylenglycoldimethacrylat und
   1,5-2,5 Gew.-% 2-Hydroxyethylmethacrylat,
   wobei sich die Gewichtsangaben auf das Gesamtgewicht des Polymers beziehen, und
c) mindestens einem weiteren fixierenden Polymer gewählt aus der Gruppe der nichtionischen und/oder kationischen Polymere.

Die Aufgabe wird ferner gelöst durch die Verwendung eines oder mehrerer nichtionischer, verdickende Polymere, welche gewählt werden aus der Gruppe der Verbindungen der Cellulosen, der Cellulosederivate und Guar Gummis, zur Reduzierung von schuppenartigen Rückständen am Haar, die beim Auskämmen von Stylingmitteln enthaltend nichtionische und/oder kationische fixierende Polymere sowie kationische Polymere , welche gebildet werden aus
31-40 Gew.-% 2-(N, N-dimethylamino)ethylmethacrylat,
52-62 Gew.-% Ethylacrylat,
2,5-5 Gew.-% Beheneth-25 methacrylat,
2-7 Gew.-% eines Ethers aus Allylalkohol und einem Polyethylenglycol/Polypropylenglycol-Ether mit durchschnittlich 30 Ethylenglycol und durchschnittlich 4 Polypropylenglycol-Einheiten,
0,05-0,2 Gew.-% Triethylenglycoldimethacrylat und
1,5-2,5 Gew.-% 2-Hydroxyethylmethacrylat,
wobei sich die Gewichtsangaben auf das Gesamtgewicht des Polymers beziehen, entstehen.

Die erfindungsgemäßen Stylingmittel erzeugen auf dem Haar einen klaren, feuchtigkeitsunempfindlichen, festigenden, stabilen und nicht klebrigen Film der sich leicht durch handelsübliche Haarshampoos auswaschen lässt.

Dabei ist es erfindungsgemäß vorteilhaft, wenn die nichtionischen, verdickenden Polymere der Gruppe a) in einer Gesamtkonzentration von 0,1 bis 10 Gewichts-% und erfindungsgemäß bevorzugt, wenn die nichtionischen Polymere der Gruppe a) in einer Gesamtkonzentration von 0,5 bis 5 Gewichts -%, jeweils bezogen auf das Gesamtgewicht des Stylingmittels in diesem enthalten sind.

Erfindungsgemäß vorteilhafte Ausführungsformen der vorliegenden Erfindung sind auch dadurch gekennzeichnet, dass das kationische Polymer b) in einer Gesamtkonzentration von 0,1 bis 10 Gewichts-% und bevorzugt in einer Gesamtkonzentration von 0,5 bis 5 Gewichts-%, jeweils bezogen auf das Gesamtgewicht des Stylingmittels in diesem enthalten ist.

Erfindungsgemäß vorteilhafte Ausführungsformen der vorliegenden Erfindung sind ferner dadurch gekennzeichnet, dass die nichtionischen und/oder kationischen fixierenden Polymeren der Gruppe c) in einer Gesamtkonzentration von 0,1 bis 10 Gewichts-% und bevorzugt in einer Gesamtkonzentration von 0,5 bis 5 Gewichts-%, jeweils bezogen auf das Gesamtgewicht des Stylingmittels in diesem enthalten ist.

Als nichtionische, verdickende Polymere a), welche gewählt werden aus der Gruppe der Verbindungen der Cellulosen, der Cellulosederivate und Guar Gummis, werden erfindungsgemäß bevorzugt Hydroxyethylcellulose (CAS 9004-62-0, z.B. Natrosol), Hydroxypropylcellulose (CAS 9004-64-2, z.B. Klucel HF), Hydroxymethylpropylcellulose (CAS 9004-65-3, z.B. Methocel) und Guar Gummis (INCI Cyamopsis Tetragonoloba (Guar) Gum, CAS 9000-30-0) eingesetzt. Dabei wird erfindungsgemäß besonders bevorzugt Grindsted Guar 178, Prod.Nr.129732 von Fa. Danisco eingesetzt.

Das kationische Polymer b), wird erfindungsgemäß bevorzugt gebildet aus
34-36 Gew.-% 2-(N, N-dimethylamino)ethylmethacrylat,
55-57 Gew.-% Ethylacrylat,
2,5-3,5 Gew.-% Beheneth-25 methacrylat,
3,5-5 Gew.-% eines Ethers aus Allylalkohol und einem Polyethylenglycol/Polypropylenglycol-Ether mit durchschnittlich 30 Ethylenglycol und
durchschnittlich 4 Polypropylenglycol-Einheiten,
0,1-0,15 Gew.-% Triethylenglycoldimethacrylat und
2 Gew.-% 2-Hydroxyethylmethacrylat,
wobei sich die Gewichtsangaben auf das Gesamtgewicht des Polymers beziehen.

Die fixierenden nichtionischen und/oder kationischen Polymere der Gruppe c) können erfindungsgemäß vorteilhaft aus der Liste der folgenden Verbindungen gewählt werden:

Fixierende kationische Polymere:
Copolymere von aminsubstituierten Vinylmonomeren und nicht aminsubstituierten, nicht kationischen Monomeren. Aminsubstituierte Vinylmonomere sind z.B. Dialkylaminoalkylacrylat, Dialkylaminoalkylmethacrylat, Monoalkylaminoalkylacrylat, Monoalkylaminoalkyl-methacrylat, wobei die Alkylgruppen dieser Monomere vorzugsweise niedere Alkylgruppen wie z.B. C1-C7- Alkylgruppen, besonders bevorzugt C1- C3-Alkylgruppen sind.
Geeignete ammoniumsubstituierte Vinylmonomere sind z.B. Trialkylmethacryloxyalkylammonium, Trialkylacryloxyalkylammonium, Dialkyldiallylammonium und quarternäre Vinylammoniummonomere mit cyclischen, kationische Stickstoffe enthaltenden Gruppen wie Pyridinium oder Imidazolium, z.B. Alkylvinylpyridinium oder Alkylvinylimidazolium Salze. Die Alkylgruppen dieser Monomere sind vor-zugsweise niedere Alkylgruppen wie z.B. C1-C7-Alkylgruppen, besonders bevorzugt C1- C3 -Alkylgruppen. Geeignete Polymere sind unter den Bezeichnungen Poly-quaternium beschriebenen Polymere wie quaternisierte Copolymere von Vinylimidazol, Vinylpyrrolidon und/oder Vinylcaprolactam (Polyquaternium-16, -44 oder -46), quaternisiertes Vinylpyrrolidon/-Dimethylaminoethylmethacrylat Copolymer (Polyquaternium-11), Homo- und Copolymere von Dimethyldiallylammoniumchlorid (Polyquaternium-6, -7 oder-22), quaternisierte Hydroxy-ethylcellulose (Polyquaternium-10) oder quaternisierte Guarderivate.
Nicht aminsubstituierte, nichtkationische Comonomere sind z.B. Acrylamid, Methacrylamid, Alkyl- und Dialkylacrylamid, Alkyl- und Dialkylmethacrylamid, Acrylacrylat, Alkylmethacrylat, Vinylcaprolacton, Vinylpyrrolidon, Vinylester, Vinylalkohol, Maleinsäreanhydrid, Propylenglycol oder Ethylenglycol, wobei die Alkylgruppen dieser Monomere vorzugsweise niedere Alkylgruppen wie z.B. C1-C7- Alkylgruppen, besonders bevorzugt C1- C3-Alkylgruppen sind.
Weitere kationische Polymere sind: quaternisiertes Vinylpyrrolidon/-Dialkylaminoalkylmethacrylat Copolymere, insbesondere quaternisiertes Vinylpyrrolidon/Dimethylaminoethylmethylacrylat Copolymer (INCI: Polyquaternium-11), z.B. Gafquat 755 von ISP oder Luviquat PQ-11 von BASF oder quaternisiertes Vinylpyrrolidon/Dimethylaminopropylmethacrylat/Lauryl Dimethylaminopropylmethacrylat Copolymer (INCI: Polyquaternium-55), z.B. Styleze W-20 von ISP; Methylvinylimidazoliumchlorid/Vinylpyrrolidon Copolymer (INCI: Polyquaternium-16), z.B. die Luviquat Typen FC 370, FC 550, FC 905 oder HM552 von BASF; Methylvinylimidazolium Methylsulfat/Vinylpyrrolidon Copolymer (INCI: Polyquaternium-44), z.B. Luviquat Care oder Luviquat MS 370 von BASF; oder das Copolymer aus Vinylcaprolactam, Vinylpyrrolidon und quaternisiertem Vinylimidazol (INCI Polyquaternium-46), z.B. Luviquat Hold von BASF.

Fixierende nichtionisch Polymere:
Geeignete nichtionische Polymere sind z.B. Homopolymere des Vinylpyrrolidons, z.B. Luviskol K von BASF oder Homopolymere des N-Vinylformamids z.B. PVF von National Starch.
Weitere geeignete Polymere sind Copolymereisate aus Vinylpyrrolidon und Vinylacetat z.B. Luviskol VA Typen von BASF, Terpolymere aus Vinylpyrrolidon, Vinylacetat und Propionat wie z.B. Luviskol VAP von BASF, Polyvinylcaprolactam, Polyvinylamide und deren Salze sowie Copolymere aus Vinylpyrrolidon und Dimethylaminoethylmethacrylat, Terpolymere aus Vinylcaprolactam, Vinylpyrrolidon und Dimethylaminomethacrylat; Polysiloxane und dergleichen mehr.

Erfindungsgemäß besonders bevorzugt werden als nichtionische und/oder kationische fixierende Polymere Methylvinylimidazoliumchlorid/Vinylpyrrolidon Copolymer (Polyquaternium-16) und/oder Vinylpyrrolidon/Vinylacetat Copolymer eingesetzt.

Das erfindungsgemäß vorteilhaft geeignete Lösungsmittel ist Wasser.
Erfindungsgemäß vorteilhaft geeignete Lösungsmittel sind außerdem organische Lösungsmittel wie aliphatische Alkohole mit C1-4 Kohlenstoffatomen oder Gemische daraus mit Wasser. Es können auch andere Lösungsmittel eingesetzt werden wie unverzweigte oder verzweigte Kohlenwasserstoffe wie Pentan, Hexan, Isopentan und zyklische Kohlenwasserstoffe wie Cyclopentan und Cyclohexan.
Mögliche wasserlösliche Lösungsmittel sind Glycerin, Ethylenglycol und Propylenglycol.

In einer erfindungsgemäß bevorzugten Ausführungsform der vorliegenden Erfindung enthält das Stylingmittel (die Zubereitung) Alkohol in einer Menge von 0,5 bis 25 Gewichts-%, bezogen auf das Gesamtgewicht der Zubereitung.

Optionale herkömmliche Additive können ebenfalls in die Formulierung einbezogen sein, um der Zusammensetzung bestimmte Modifizierungseigenschaften zu verleihen: dies sind Silikone oder Silikonderivate, Benetzungsmittel, Feuchthaltemittel, Weichmacher wie Glycerin, Glykol und Phthalester und - ether, erweichende Mittel, Riechstoffe und Parfüms, UV, Absorber, Farbstoffe , Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner.
Diese Additive sind im allgemeinen in einer Konzentration von etwa 0,01% bis 10 Gew.% bezogen auf das Gesamtgewicht der Zusammensetzung vorhanden.

Die erfindungsgemäße Zubereitung enthält vorteilhafter Weise einen oder mehrere Konditionierer. Erfindungsgemäß bevorzugte Konditionierer sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, Skin-Conditioning Agents-Emollient, Skin-Conditioning Agents-Humactant, Skin-Conditioning Agents-Miscellaneous, Skin-Conditioning Agents-Occlusive und Skin Protectans aufgeführt sind sowie alle in der EP 0934956 (S.11-13) unter water soluble conditioning agent und oil soluble conditioning agent aufgeführten Verbindungen. Weitere erfindungsgemäß vorteilhafte Konditionierer stellen beispielsweise die nach der internationalen Nomenklatur für kosmetische Inhaltsstoffe (INCI) als Polyquaternium benannten Verbindungen dar (insbesondere Polyquaternium-1 bis Polyquaternium-56).

Als erfindungsgemäß bevorzugte Konditionierer aus der Gruppe der Polyquaternium-Verbindungen können dabei beispielsweise Polyquaternium-4 oder Polyquaternium-46 eingesetzt werden.

In einer erfindungsgemäß bevorzugten Ausführungsform der vorliegenden Erfindung liegt das Stylingmittel (die Zubereitung) als sprühbare oder schäumbare Zubereitung vor. Diese wir erfindungsgemäß vorteilhaft zusammen mit einem Treibgas in einem Druckgasbehälter (Sprühdose) aufbewahrt und aus dieser heraus angewendet. In dieser Ausführungsform enthält die erfindungsgemäße Zubereitung vorteilhaft 5 bis 20 Gewichts-% Treibgas.

Erfindungsgemäß besonders vorteilhafte Treibgase sind Dimethylether, Propan, Isopropan, Isobutan und n-Butan sowie deren Mischungen.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Eine besonders bevorzugte Ausführungsform der erfindungsgemäßen Haarfestiger ist ihr Vorliegen in Form einer wässrig und/oder alkoholischen Lösung.

Die erfindungsgemäßen Haarfestiger werden erfindungsgemäß vorteilhaft in einer Pumpschäumer- oder Aerosolverpackung als Schaumspender verpackt (aufbewahrt), aus der heraus sie vorteilhaft angewendet werden können.

Auch sind Schaumspender auf Pumpschäumer- oder Aerosolverpackungsbasis, welche einen erfindungsgemäßen Haarfestiger enthalten, erfindungsgemäß.

Erfindungsgemäß ist weiterhin die Verwendung erfindungsgemäßen Haarfestigers als Schaumfestiger.

In einer weiteren, erfindungsgemäß besonders bevorzugten Ausführungsform der vorliegenden Erfindung liegt das Stylingmittel (die Zubereitung) als Gel vor.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Rezepturbeispiele :

| | **1** | **2** | **3** | **4** | **5** |
|---|---|---|---|---|---|
| VP/VA Copolymer | 6,6 | 10,0 | 10,0 | 6,6 | 6,6 |
| Polyquaternium-4 | 1,0 | 0,5 | 0,5 | 1,0 | 1,0 |
| PQ-16 | 1,0 | - | - | 1,0 | - |
| Cationic thickening polymer EX 797 (Fa. Noveon) | 4,0 | 2,0 | 0,5 | 3,0 | 1,0 |
| Hydroxymethylpropylcellulose | 0,5 | - | 2,0 | - | 0,5 |
| Hydroxyethylcellulose | - | 0,2 | - | - | - |
| Guar Gum | - | 0,5 | - | 1,0 | - |
| Ethanol | - | - | - | 15,0 | 20,0 |
| Cetrimoniumchlorid | 0,3 | 0,3 | 0,3 | 0,3 | 0,3 |
| Silikonöl* | 0,07 | - | - | - | 0,07 |
| Glycerin | 5,0 | - | 2,0 | - | - |
| Parfüm, Lösungsvermittler, pH-Einstellung, Lichtschutz, Konservierung | q.s. | q.s. | q.s. | q.s. | q.s. |
| Wasser | ad 100 | ad 100 | ad 100 | ad 100 | ad 100 |

| | | | | | |
|---|---|---|---|---|---|
| (*) insbesondere PEG/PPG-20/6 Dimethicone von Goldschmidt | | | | | |

| | **7** | **8** | **9** | **10** |
|---|---|---|---|---|
| VP/VA Copolymer | - | - | 6,0 | - |
| PVP | 1,0 | 4,0 | - | 3,0 |
| PQ-4 | 3,0 | - | 1,0 | 0,5 |
| Cationic thickening polymer EX 797 (Fa. Noveon) | 3,0 | 0,5 | 2,0 | 2,0 |
| Hydroxyethylcellulose | - | 3,0 | 1,0 | - |
| Guar Gum | 0,5 | - | - | 1,0 |
| Ethanol | 5,0 | 15,0 | - | 5,0 |
| Cetrimoniumchlorid | 0,3 | 0,3 | 0,2 | 0,2 |
| Silikonöl* | 0,07 | 0,07 | - | - |
| Glycerin | 10,0 | 2,0 | - | - |
| Parfüm, Lösungsvermittler, pH-Einstellung, Lichtschutz, Konservierung | q.s. | q.s. | q.s | q.s |
| Isobutan/Propan/Butan | - | - | 9,0 | 9,0 |
| Wasser | ad 100 | ad 100 | ad 100 | ad 100 |

| | | | | |
|---|---|---|---|---|
| (*) insbesondere PEG/PPG-20/6 Dimethicone von Goldschmidt | | | | |

## Patentansprüche

1. Stylingmittel enthaltend eine Kombination aus
a) einem oder mehreren nichtionischen, verdickenden Polymeren, welche gewählt werden aus der Gruppe der Verbindungen der Cellulosen, der Cellulosederivate und der Guar Gummis,
b) einem kationischen Polymer, welches gebildet wird aus
31-40 Gew.-% 2-(N, N-dimethylamino)ethylmethacrylat,
52-62 Gew.-% Ethylacrylat,
2,5-5 Gew.-% Beheneth-25 methacrylat,
2-7 Gew.-% eines Ethers aus Allylalkohol und einem
Polyethylenglycol/Polypropylenglycol-Ether mit durchschnittlich 30 Ethylenglycol und durchschnittlich 4 Polypropylenglycol-Einheiten,
0,05-0,2 Gew.-% Triethylenglycoldimethacrylat und
1,5-2,5 Gew.-% 2-Hydroxyethylmethacrylat,
wobei sich die Gewichtsangaben auf das Gesamtgewicht des Polymers beziehen, und
c) mindestens einem weiteren fixierenden Polymer gewählt aus der Gruppe der nichtionischen und/oder kationischen Polymere.

2. Verwendung eines oder mehrerer nichtionischer, verdickende Polymere, welche gewählt werden aus der Gruppe der Verbindungen der Cellulosen, der Cellulosederivate und Guar Gummis, zur Reduzierung von schuppenartigen Rückständen am Haar, die beim Auskämmen von Stylingmitteln enthaltend nichtionische und/oder kationische fixierende Polymere sowie kationische Polymere , welche gebildet werden aus
31-40 Gew.-% 2-(N, N-dimethylamino)ethylmethacrylat,
52-62 Gew.-% Ethylacrylat,
2,5-5 Gew.-% Beheneth-25 methacrylat,
2-7 Gew.-% eines Ethers aus Allylalkohol und einem
Polyethylenglycol/Polypropylenglycol-Ether mit durchschnittlich 30 Ethylenglycol und durchschnittlich 4 Polypropylenglycol-Einheiten,
0,05-0,2 Gew.-% Triethylenglycoldimethacrylat und
1,5-2,5 Gew.-% 2-Hydroxyethylmethacrylat,
wobei sich die Gewichtsangaben auf das Gesamtgewicht des Polymers beziehen, entstehen.

3. Stylingmittel nach Anspruch 1 oder Verwendung nach Anspruch 2, **dadurch gekennzeichnet, dass** die nichtionischen, verdickenden Polymere der Gruppe a) in einer Gesamtkonzentration von 0,1 bis 10 Gewichts-%, bezogen auf das Gesamtgewicht des Stylingmittels in diesem enthalten sind.

4. Stylingmittel oder Verwendung nach einem der vorhergehenden Ansprüche, **dadurch gekennzeichnet, dass** das kationische Polymer b) in einer Gesamtkonzentration von 0,1 bis 10 Gewichts-%, bezogen auf das Gesamtgewicht des Stylingmittels in diesem enthalten ist.

5. Stylingmittel nach einem der vorhergehenden Ansprüche, **dadurch gekennzeichnet, dass** die nichtionischen und/oder kationischen fixierenden Polymeren der Gruppe c) in einer Gesamtkonzentration von 0,1 bis 10 Gewichts-%, bezogen auf das Gesamtgewicht des Stylingmittels in diesem enthalten ist.

6. Stylingmittel nach einem der vorhergehenden Ansprüche, **dadurch gekennzeichnet, dass** als nichtionische und/oder kationische fixierende Polymere Methylvinylimidazoliumchlorid/Vinylpyrrolidon Copolymer (Polyquaternium-16) und/oder VinylpyrrolidonNinylacetat Copolymer eingesetzt werden.

7. Stylingmittel oder Verwendung nach einem der vorhergehenden Ansprüche, **dadurch gekennzeichnet, dass** die Zubereitung einen Alkoholgehalt von 0,5 bis 25 Gewichts-%, bezogen auf das Gesamtgewicht der Zubereitung, aufweist.

8. Stylingmittel oder Verwendung nach einem der vorhergehenden Ansprüche, **dadurch gekennzeichnet, dass** die Zubereitung als Gel vorliegt.

9. Stylingmittel oder Verwendung nach einem der vorhergehenden Ansprüche, **dadurch gekennzeichnet, dass** die Zubereitung als sprühbare Zubereitung vorliegt.

10. Stylingmittel oder Verwendung nach Anspruch 9, **dadurch gekennzeichnet, dass** die Zubereitung 5 bis 20 Gewichts-% Treibgas enthält.

## Claims

1. Styling composition comprising a combination of
a) one or more nonionic, thickening polymers which are selected from the group of the compounds of the celluloses, the cellulose derivatives and the guar gums,
b) a cationic polymer which is formed from
31-40% by weight of 2-(N,N-dimethylamino)ethyl methacrylate,
52-62% by weight of ethyl acrylate,
2.5-5% by weight of beheneth-25 methacrylate,
2-7% by weight of an ether of allyl alcohol and a polyethylene glycol/polypropylene glycol ether with, on average, 30 ethylene glycol and, on average, 4 polypropylene glycol units,
0.05-0.2% by weight of triethylene glycol dimethacrylate and
1.5-2.5% by weight of 2-hydroxyethyl methacrylate,
where the weight data refer to the total weight of the polymer, and
c) at least one further fixing polymer selected from the group of the nonionic and/or cationic polymers.

2. Use of one or more nonionic, thickening polymers which are selected from the group of the compounds of the celluloses, the cellulose derivatives and guar gums, for reducing flaky residues on the hair which arise when combing out styling compositions comprising nonionic and/or cationic fixing polymers and also cationic polymers which are formed from
31-40% by weight of 2-(N,N-dimethylamino)ethyl methacrylate,
52-62% by weight of ethyl acrylate,
2.5-5% by weight of beheneth-25 methacrylate,
2-7% by weight of an ether of allyl alcohol and a polyethylene glycol/polypropylene glycol ether with, on average, 30 ethylene glycol and, on average, 4 polypropylene glycol units,
0.05-0.2% by weight of triethylene glycol dimethacrylate and
1.5-2.5% by weight of 2-hydroxyethyl methacrylate,
where the weight data refer to the total weight of the polymer.

3. Styling composition according to Claim 1 or use according to Claim 2, **characterized in that** the nonionic, thickening polymers of group a) are present in this in a total concentration of from 0.1 to 10% by weight, based on the total weight of the styling composition.

4. Styling composition or use according to one of the preceding claims, **characterized in that** the cationic polymer b) is present in this in a total concentration of from 0.1 to 10% by weight, based on the total weight of the styling composition.

5. Styling composition according to one of the preceding claims, **characterized in that** the nonionic and/or cationic fixing polymers of group c) are present in this in a total concentration of from 0.1 to 10% by weight, based on the total weight of the styling composition.

6. Styling composition according to one of the preceding claims, **characterized in that** methylvinylimidazolium chloride/vinylpyrrolidone copolymer (polyquaternium-16) and/or vinylpyrrolidone/vinyl acetate copolymer are used as nonionic and/or cationic fixing polymers.

7. Styling composition or use according to one of the preceding claims, **characterized in that** the preparation has a alcohol content of from 0.5 to 25% by weight, based on the total weight of the preparation.

8. Styling composition or use according to one of the preceding claims, **characterized in that** the preparation is in the form of a gel.

9. Styling composition or use according to one of the preceding claims, **characterized in that** the preparation is in the form of a sprayable preparation.

10. Styling composition or use according to Claim 9, **characterized in that** the preparation comprises 5 to 20% by weight of propellant gas.

## Revendications

1. Combinaison de polymères pour des compositions de mise en forme des cheveux, contenant une combinaison
a) d'un ou de plusieurs polymères non ioniques, épaississants qui sont choisis parmi le groupe des composés de celluloses, des dérivés de la cellulose et des gommes de guar,
b) d'un polymère cationique, qui est formé à partir de
31-40 % en poids de méthacrylate de 2-(N,N-diméthylamino)éthyle,
52-62 % en poids d'acrylate d'éthyle,
2,5-5 % en poids de méthacrylate de béhéneth-25,
2-7 % en poids d'un éther fait d'un alcool allylique et d'un éther polyéthylèneglycol/polypropylèneglycol ayant en moyenne 30 unités d'éthylèneglycol et en moyenne 4 unités de polypropylèneglycol,
0,05-0,2 % en poids de diméthacrylate de triéthylèneglycol et
1,5-2,5 % en poids de méthacrylate de 2-hydroxyéthyle,
les indications en poids se rapportant au poids total du polymère et
c) d'au moins un polymère fixant supplémentaire choisi parmi le groupe des polymères non ioniques et/ou cationiques.

2. Utilisation d'un ou de plusieurs polymères non ioniques, épaississants qui sont choisis parmi le groupe des composés de celluloses, des dérivés de la cellulose et des gommes de guar, en vue de la réduction des résidus similaires aux pellicules sur la chevelure, qui se forment lors du peignage à l'aide de compositions de mise en forme des cheveux contenant des polymères non ioniques et/ou cationiques fixants ainsi que des polymères cationiques, qui sont formés à partir de
31-40 % en poids de méthacrylate de 2-(N,N-diméthylamino)éthyle,
52-62 % en poids d'acrylate d'éthyle,
2,5-5 % en poids de méthacrylate de béhéneth-25,
2-7 % en poids d'un éther fait d'un alcool allylique et d'un éther polyéthylèneglycol/polypropylèneglycol ayant en moyenne 30 unités d'éthylèneglycol et en moyenne 4 unités de polypropylèneglycol,
0,05-0,2 % en poids de diméthacrylate de triéthylèneglycol et
1,5-2,5 % en poids de méthacrylate de 2-hydroxyéthyle,
les indications en poids se rapportant au poids total du polymère.

3. Composition de mise en forme des cheveux selon la revendication 1, ou utilisation selon la revendication 2, **caractérisé en ce que** les polymères non ioniques, épaississants du groupe a) sont contenus dans celui-ci dans une concentration globale de 0,1 à 10 % en poids, par rapport au poids total de la composition de mise en forme des cheveux.

4. Composition de mise en forme des cheveux ou utilisation selon l'une quelconque des revendications précédentes, **caractérisé en ce que** le polymère cationique b) est contenu dans celui-ci dans une concentration globale de 0,1 à 10 % en poids, par rapport au poids total de la composition de mise en forme des cheveux.

5. Composition de mise en forme des cheveux selon l'une quelconque des revendications précédentes, **caractérisé en ce que** les polymères non ioniques et/ou cationiques fixant du groupe c) sont contenus dans celui-ci dans une concentration globale de 0,1 à 10 % en poids, par rapport au poids total de la composition de mise en forme des cheveux.

6. Composition de mise en forme des cheveux selon l'une quelconque des revendications précédentes, **caractérisé en ce que** l'on utilise, en tant que polymère non ionique et/ou cationique, le copolymère chlorure de méthylvinylimidazolium/vinylpyrrolidone (polyquaternium-16) et/ou le copolymère vinylpyrrolidone/acétate de vinyle.

7. Composition de mise en forme des cheveux ou utilisation selon l'une quelconque des revendications précédentes, **caractérisé en ce que** la préparation présente une teneur en alcool de 0,5 à 25 % en poids, par rapport au poids total de la préparation.

8. Composition de mise en forme des cheveux ou utilisation selon l'une quelconque des revendications précédentes, **caractérisé en ce que** la préparation est présente sous forme de gel.

9. Composition de mise en forme des cheveux ou utilisation selon l'une quelconque des revendications précédentes, **caractérisé en ce que** la préparation est présente en tant que préparation pulvérisable.

10. Composition de mise en forme des cheveux ou utilisation selon la revendication 9, **caractérisé en ce que** la préparation contient 5 à 20 % en poids de gaz propulsant.
